# EUROPEAN PATENT SPECIFICATION

(11) **EP 4 577 640 B1**
(45) Date of publication and mention of the grant of the patent: **06.05.2026**
(21) Application number: 23935879.9
(22) Date of filing: 30.10.2023
(51) Int. Cl.: C07C 235/38, A61P 19/04, A61K 35/32, C12N 5/0775, C12N 5/077, A61K 31/196

(54) **COMPOSITION COMPRISING AVENANTHRAMIDE C FOR INDUCING DIFFERENTIATION OF MESENCHYMAL STEM CELLS INTO CHONDROCYTES AND METHOD OF USING THE SAME**
ZUSAMMENSETZUNG MIT AVENANTHRAMID C ZUR INDUKTION DER DIFFERENZIERUNG MESENCHYMALER STAMMZELLEN IN CHONDROZYTEN UND VERFAHREN ZUR VERWENDUNG DAVON
COMPOSITION COMPRENANT DE L'AVENANTHRAMIDE C POUR INDUIRE LA DIFFÉRENCIATION DE CELLULES SOUCHES MÉSENCHYMATEUSES EN CHONDROCYTES ET SON PROCÉDÉ D'UTILISATION

(43) Date of publication of application: 02.07.2025
(73) Proprietor: Medeze Treasury Ptd. Ltd, 117406 Singapore (SG)
(72) Inventor: KHEMARANGSAN, Veerapol, Singapore 117406 (SG)
(74) Representative: Curell Suñol S.L.P.
(86) International application number: PCT/IB2023/060888
(87) International publication number: WO 2025/093898

(56) References cited:
- WO-A1-2012/126824
- CN-A- 111 534 483
- KR-A- 20210 068 918
- FU RONG ET AL: "Avenanthramide C induces cellular senescence in colorectal cancer cells via suppressing [beta]-catenin-mediated the transcription of miR-183/96/182 cluster", BIOCHEMICAL PHARMACOLOGY, ELSEVIER, US, vol. 199, 28 March 2022 (2022-03-28), XP087032662, ISSN: 0006-2952, [retrieved on 20220328], DOI: 10.1016/J.BCP.2022.115021
- WEI-CHUAN CHEN ET AL: "Compare the effects of chondrogenesis by culture of human mesenchymal stem cells with various type of the chondroitin sulfate C", JOURNAL OF BIOSCIENCE AND BIOENGINEERING, ELSEVIER, AMSTERDAM, NL, vol. 111, no. 2, 3 October 2010 (2010-10-03), pages 226 - 231, XP028136638, ISSN: 1389-1723, [retrieved on 20101011], DOI: 10.1016/J.JBIOSC.2010.10.002

## Description

### TECHNICAL FIELD

The present invention is generally related to an application of Avenanthramide C (Avn C) as an inducer of chondrocyte differentiation in Mesenchymal Stem Cells (MSCs). More particularly, the presently disclosed embodiments are related to the differentiation of mesenchymal stem cells (MSCs) into chondrocytes, a composition comprising avenanthramide c (Avn C), and a method of using the same.

### BACKGROUND

In the context of aging populations, the decrease in operational chondrocytes, identified by their limited regenerative ability, significantly contributes to the gradual worsening of joint discomfort over an extended timeframe. Chondrocytes, essential cells responsible for producing and releasing various components of the extracellular matrix, encompassing diverse types of collagens, are vital for upholding cartilage flexibility. The introduction of chondrocyte replacement therapy has the potential to notably improve the quality of life for individuals grappling with joint-related concerns.

Mesenchymal Stem Cells (MSCs), possessing the capacity to differentiate into various cell lineages, including chondrocytes, have generated significant interest in the development of MSC-based therapies to restore cartilage. By utilizing the inherent characteristics of a patient's MSCs and orchestrating their directed differentiation toward the chondrocyte lineage, this inventive approach offers a secure alternative that effectively avoids potential risks linked to immune system rejection of cellular grafts.

Mesenchymal Stem Cells (MSCs) are adult stem cells derived from diverse tissues, holding great potential for regenerative cartilage therapies due to their multipotent differentiation abilities. However, a practical and dependable method for inducing chondrocyte differentiation in MSCs is necessary. Current methods lack optimal effectiveness, prompting the requirement for a novel inducer with superior results.

Avenanthramides (Avn), polyphenolic compounds predominantly found in oats, have gained attention due to their distinctive biological properties, including antioxidant, antiproliferative, anti-histamine, and anti-inflammatory effects.

Korean patent application KR20210091080A1 titled "Pharmaceutical composition for treating or preventing arthritis" and filed by RYU JE HWANG et al. discloses the use of Avn-C to decrease the expression of cartilage matrix-degrading enzyme and cartilage degeneration inducers of chondrocytes for the treatment or prevention of arthritis. Document FU RONG ET AL: 2022 (DOI: 10.1016/j.bcp.2022.115021) relates to the senescence-inducing effect of AVN C in colorectal cancer cells.

Further, a non-patent literature titled "Avenanthramide C as a novel candidate to alleviate osteoarthritic pathogenesis" published by Thanh-Tam Tran et al. discloses the use of Avenanthramide-C for the prevention of osteoarthritis (OA) progression and to prevent the expression of matrix-metalloproteinases (MMPs) in OA pathogenesis. This non-patent literature also highlights that the administration of Avn-C did not affect the expression of inflammatory mediators (Ptgs2 and Nos) or anabolic factors (Col**2**a**1**, Aggrecan, and Sox9) in OA pathogenesis.

Another non-patent literature titled "Avenanthramides Prevent Osteoblast and Osteocyte Apoptosis and Induce Osteoclast Apoptosis in Vitro in an Nrf2-Independent Manner" published by Gretel G. Pellegrini et al. discloses the application of Avenanthramide to enhance osteogenic differentiation by increasing the expression of COL1A. The study demonstrates that the Avenanthramides exert their anti-apoptotic effects on osteoblastic cells and their pro-apoptotic effects on osteoclasts.

Another, recent research conducted by Trans et al. sheds light on the inhibitory effects of Avenanthramide C (Avn C) on cartilage degradation. The study revealed that Avn C effectively suppressed the expression of catabolic factors, specifically MMP-**3**, -12, and -13, induced by IL-**1**β in mouse articular chondrocytes. However, Avn C did not impact the IL-**1**β-mediated reduction of mature factors such as Collagen 2 (COL2A1), a major component of the cartilage extracellular matrix (ECM), or SOX9, a crucial transcription factor involved in collagen 2 regulation. Notably, Avn C was applied to inflammatory responses of fully differentiated chondrocytes within mature cartilage during this investigation.

This specification recognizes there is a need for a novel application of Avenanthramide C (Avn C) as a potent inducer of chondrocyte differentiation in Mesenchymal Stem Cells (MSCs), establishing a more effective approach in the field. MSCs, which are adult stem cells derived from various tissues such as bone marrow, adipose tissue, and umbilical cord tissue, possess remarkable versatility and can differentiate into multiple cell types.

The above-mentioned shortcomings, disadvantages, and problems are addressed herein and which will be understood by reading and studying the following specification.

### SUMMARY

The various embodiments herein provide differentiation of mesenchymal stem cells (MSCs) into chondrocytes, with a composition according to claim 1, and the method of using the same.

An aspect of the present disclosure relates to a novel application of Avenanthramide C (Avn C) as a potent inducer of chondrocyte differentiation in Mesenchymal Stem Cells (MSCs), establishing a more effective approach in the field. MSCs, which are adult stem cells derived from various tissues such as bone marrow, adipose tissue, and umbilical cord tissue, possess remarkable versatility and can differentiate into multiple cell types. This unique characteristic positions MSCs as an invaluable resource for regenerative cartilage applications.

Through the utilization of Avn C treatments, MSCs demonstrated an impressive capacity to undergo chondrocyte differentiation, evident through a heightened expression of key chondrogenic extracellular matrix (ECM) factors, most notably collagen type 2 (COL2A1), specific to chondrocytes. The synergistic effect of Avn C combined with Chondroitin Sulfate (CS) notably elevated the transcriptionally active state of SOX9 expression, a critical regulator of chondrogenesis.

In one aspect, employing Avn C at concentrations ranging from 10nM to 50uM (50,000 nM), administered concurrently with 300-700 ug/mL of Chondroitin Sulfate, particularly 500 ug/mL, as highly effective differentiation factors facilitating the transformation of MSCs into chondrocyte-like cells. With this optimized formulation, we observed the metamorphosis of MSCs, leading to the adoption of chondrocyte-like characteristics, as evidenced by substantial changes in mRNA gene expressions (ACAN, LRP1, SOX9), cellular protein levels (COL2A1, SOX9, LRP1), and functional levels, exemplified by the secretion of extracellular matrix proteins (FSTL-1, BGH3, CO**1**A**1**, CO**1**A**2**, and CO3A1). These outcomes firmly establish Avn C as a highly effective differentiation factor, paving the way for its practical application in the field of chondrocyte differentiation from MSCs.

An aspect of the present disclosure relates to a composition for inducing differentiation of mesenchymal stem cells (MSCs) into chondrocytes, according to claim 1. According to the invention, the term "composition" refers to a medium or hydrogel according to claim 1 used to induce the stem cell into chondrocytes and the term "chondrocyte-like cells" refers to 2-dimensional manner chondrocyte-like cells.

In an aspect, the Avenanthramide C (Avn C) is present at a concentration in the range of **10** nM - **50,000** nM.

In an aspect, the Avenanthramide C (Avn C) is preferably present at the concentration of 50 - 300 nM.

The composition according to the invention further includes chondroitin sulfate (CS) as a bioactive factor.

In an aspect, the composition further includes chondroitin sulfate (CS) as a bioactive factor.

In an aspect, the chondroitin sulfate (CS) is present at the concentration in the range of **300** - 700 µg/mL, particularly 500 ug/mL.

In an aspect, mesenchymal stem cells are adipose-derived mesenchymal stem cells (aMSCs).

In an aspect, chondrocytes are 2-dimensional manner chondrocyte-like cells.

In an aspect, the composition is used for increasing chondrocyte markers in the chondrocytes, and the chondrocyte markers are selected from the group consisting of FSTL-1, LRP1, ACAN, COL2A, SOX9, and any combination thereof.

Another aspect of the present disclosure relates to the use of a medium for inducing *in vitro* differentiation of mesenchymal stem cells (MSCs). The medium comprises the composition having Avenanthramide C (Avn C) at a concentration in the range of 10 nM - 50,000 nM. In addition, such composition further includes chondroitin sulfate (CS) as a bioactive factor at the concentration in the range of 300 - 700 µg/mL, particularly 500 ug/mL.

Another aspect of the present disclosure relates to a method for inducing differentiation of *in vitro* cultured mesenchymal stem cells (MSCs) into chondrocytes, said method comprising a step of culturing said MSCs in a medium, according to claim 8.

In an aspect, the Avenanthramide C (Avn C) is present at a concentration in the range of 10 nM - 50,000 nM.

In an aspect, the Avenanthramide C (Avn C) is preferably present at the concentration of 50 nM - 300 nM.

In the method according to the invention, the chondroitin sulfate (CS) is present in the medium as a bioactive factor.

In the method according to the invention, chondroitin sulfate (CS) is present in the medium as a bioactive factor.

In an aspect, the chondroitin sulfate (CS) is present in the medium at the concentration in the range of 300 - 700 µg/mL, particularly 500 ug/mL.

In an aspect, the mesenchymal stem cells are adipose-derived mesenchymal stem cells (aMSCs).

In an aspect, chondrocytes are 2-dimensional manner chondrocyte-like cells.

In an aspect, chondrocyte markers of the chondrocytes are increased, and the chondrocyte markers are selected from the group consisting of FSTL-1, **LRP1,** ACAN, COL2A, SOX9, and any combination thereof.

Another aspect of the present disclosure relates to a pharmaceutical composition for use in cartilage cellular repair and regeneration.

These and other aspects of the embodiments herein will be better appreciated and understood when considered in conjunction with the following description and the accompanying drawings. It should be understood, however, that the following descriptions, while indicating the preferred embodiments and numerous specific details thereof, are given by way of illustration and not of limitation. Many changes and modifications may be made within the scope of the embodiments herein without departing from the spirit thereof, and the embodiments herein include all such modifications.

### BRIEF DESCRIPTION OF THE DRAWINGS

The other objects, features, and advantages will occur to those skilled in the art from the following description of the preferred embodiment and the accompanying drawings in which:
FIGS. 1A-1C illustrate perspective views of key aspects of *in vitro* chondrocyte differentiation process for MSCs, according to one embodiment herein.
FIGS. 2A-2D illustrate perspective views of Avenanthramides (Avn) C which exerts a potent effect on chondrogenic differentiation by significantly elevating the expression of Collagen 2 protein, according to one embodiment herein.
FIGS. 3A-3H illustrate perspective views of identification of the effective dosage range of Avenanthramide C (Avn C) induction in MSC-to-chondrocyte differentiation, in accordance with an embodiment.
FIGS. 4A-4E illustrate perspective views of the synergistic effects of Avn C with Chondroitin sulfate (CS) on SOX9 protein expression, according to one embodiment herein.
FIGS. 5A-5F illustrate perspective views of synergistic effects of Avn C and Chondroitin Sulfate (CS) on LRP1 protein expression, in accordance with an embodiment.
FIG. 6 illustrates a perspective view of the synergistic effects of Avn C and Chondroitin Sulfate (CS) on Aggrecan protein expression revealed by Alcian blue staining, according to one embodiment herein.
FIG. 7 illustrates a perspective view of mRNA expression analysis by RT-PCR: Avn C exhibits synergistic effects with Chondroitin sulfate (CS) for Aggrecan (ACAN), SOX9, and LRP1 gene expression, while maintaining COL2A1 gene expression, according to one embodiment herein.
FIG. 8 illustrates a perspective view of mRNA expression analysis of chondrocyte markers in multiple individuals' MSCs using RT-PCR, according to one embodiment herein.

Although the specific features of the embodiments herein are shown in some drawings and not in others. This is done for convenience only as each feature may be combined with any or all of the other features in accordance with the embodiments herein.

### DETAILED DESCRIPTION OF THE EMBODIMENTS HEREIN

In the following detailed description, a reference is made to the accompanying drawings that form a part hereof, and in which the specific embodiments that may be practiced is shown by way of illustration. These embodiments are described in sufficient detail to enable those skilled in the art to practice the embodiments and it is to be understood that the logical, mechanical, and other changes may be made without departing from the scope of the embodiments. The following detailed description is therefore not to be taken in a limiting sense.

The various embodiments herein provide an application of Avenanthramide C (Avn C) as a highly effective inducer of chondrocyte differentiation in MSCs. Avn C treatments exhibit superior chondrogenic potential, resulting in the upregulation of key chondrogenic extracellular matrix factors, including collagen type 2 (COL2A1). Furthermore, a synergistic effect is observed when Avn C is combined with Chondroitin Sulfate (CS), enhancing the transcriptionally active state of SOX9 expression, a pivotal regulator of chondrogenesis.

According to one embodiment herein, the formulation of the present invention comprising Avn C at concentrations ranging from 10nM to 50uM (50,000 nM), administered concurrently with 300-700ug/mL, particularly 500 ug/mL, of Chondroitin Sulfate, as an optimized and highly effective differentiation factor for MSCs, leading to their transformation into chondrocyte-like cells. The inventive formulation demonstrates significant improvements in mRNA gene expressions (ACAN, LRP1, SOX9), cellular protein levels (COL2A1, SOX9, LRP1), and functional outcomes, as evidenced by the secretion of extracellular matrix proteins (FSTL-1, BGH3, CO**1**A**1**, CO**1**A**2**, and CO3A1). These outcomes confirm the potential of Avn C as a valuable and highly effective MSC-to-chondrocyte differentiation factor for patent protection.

Also, certain terminologies and naming conventions have been used throughout this description. As one skilled in the art may appreciate, it is possible to use different names to refer to the same idea or feature, which will not limit the scope of the invention, unless specifically mentioned otherwise. The functionality of the various parts of the embodiments should be assigned importance over the names by which they are referred to, as the names by themselves are meant only for differentiation. Numerical values stated in this disclosure may be approximate or exact unless otherwise mentioned specifically and the various embodiments may deviate from the stated numbers without deviating from the intended scope.

Accordingly, one advantage of the present invention is that it provides a unique and innovative use of Avenanthramide C (Avn C) as an inducer of chondrocyte differentiation in MSCs, offering a significant advancement in the field.

Accordingly, another advantage of the present invention is that Avn C treatments demonstrate superior efficacy in promoting chondrocyte differentiation, as evidenced by the heightened expression of key chondrogenic extracellular matrix factors, particularly collagen type 2 (COL2A1).

Accordingly, another advantage of the present invention is that the combination of Avn C with Chondroitin Sulfate (CS) exhibits a synergistic effect, leading to increased expression of the critical chondrogenic regulator, SOX9, thereby enhancing the differentiation potential of MSCs.

Accordingly, another advantage of the present invention is that it provides an optimized formulation comprising specific concentrations of Avn C and Chondroitin Sulfate, providing a reliable and highly effective approach for inducing chondrocyte-like features in MSCs.

In an embodiment, the present disclosure presents Avenanthramide C (Avn C) as a novel and effective differentiation factor for inducing chondrocyte differentiation in Mesenchymal Stem Cells (MSCs). The disclosed formulation, in conjunction with Chondroitin Sulfate, offers superior outcomes in terms of gene expressions, protein levels, and functional characteristics of differentiated cells. This invention holds significant potential for practical applications in regenerative cartilage therapies and warrants patent protection.

According to an embodiment herein, the present disclosure further describes materials and methods used to implement this invention.

### Cell culture: Mesenchymal Stem Cells used in this invention are Adipose-derived Mesenchymal Stem Cells (MSCs)

All MSCs were cultured and expanded in low-glucose Dulbecco's Modified Eagle's medium (DMEM LG; Gibco #31600083), MCDB 201 (Sigma-Aldrich #M6770), and MCDB 131 (Sigma-Aldrich #M8537), in the ratio of 3:1:1. supplemented with 0.5% Human Platelet Lysates (HPL; Stemcell Technologies #06961), 1 X Insulin-Transferrin-Selenium-X (ITS-X; Gibo #51500056) or ITS+1 Liquid Media Supplement (Sigma-Aldrich #I2521), 0.2% Bovine Serum Albumin (BSA; MP Biomedicals #0219989880), 1 X Linoleic acid BSA (Sigma-Aldrich #L9530), 100 µM L-ascorbate-2-phosphate (Sigma-Aldrich #49752), 100 µM b-mercaptoethanol (Gibco #21985023), 10 ng/ml recombinant human epidermal growth factor (rhEGF; Gibco #PHG0314), **1**nM dexamethasone (Sigma-Aldrich #D4902), and 1% Penicillin-Streptomycin (pen-strep; Gibco #15140122), 250 ng/ml Amphotericin B (Amp B; Gibco #15290026) and 20 ug/ml gentamicin solution (Sigma-Aldrich #G1272).

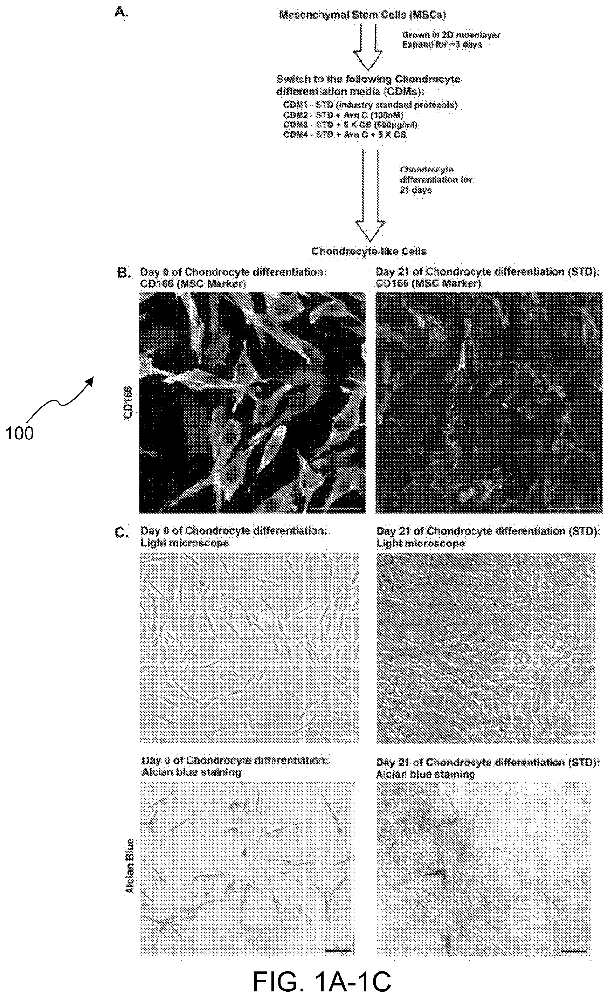

*In Vitro* Chondrogenic Differentiation: *In Vitro,* chondrogenic differentiation using monolayer culture was performed. P4 MSCs were seeded in Gelatin-coated 24 well plates at **6000** cells/cm**2**. MSCs were expanded for **3** days before switching to the following Chondrocyte differentiation media (CDM) for **21** days (FIG. **1**A).

Standard chondrocyte differentiation medium (STD) consists of DMEM Low Glucose (DMEM LG; Gibco #**31600083**), supplemented with **0.5** µg/ml hydrocortisone (Sigma-Aldrich #H0**888**), 1% Fetal bovine serum (FBS; Biowest #S**181**B), **1** X Insulin-Transferrin-Selenium-X (ITS-X; Gibo #**51500056**) or ITS+1 Liquid Media Supplement (Sigma-Aldrich #I**2521**), **10**ng/ml recombinant human TGF-beta **3** (R&D Systems **#8420-B3-025), 50**nM L-ascorbate-**2**-phosphate (Sigma-Aldrich **#49752),** 1% Penicillin-Streptomycin (pen-strep; Gibco **#15140122)** and **500**ng/ml Amphotericin B (Amp B; Gibco **#15290026).** Standard CDM (STD) will be used as a control (FIG. 1A).

Standard chondrocyte differentiation medium (STD) or CDM1 is then supplemented with **3** different treatment groups: **100** nM Avenanthramide C (Avn C; Sigma-Aldrich **#36465)** in CDM2, **500** ug/mL Chondroitin Sulfate (**5** x CS; Sigma-Aldrich #C**4384**) in CDM3, and combination of **100** nM Avenanthramide C and **500** ug/mL Chondroitin Sulfate in CDM4 (FIG. 1A).

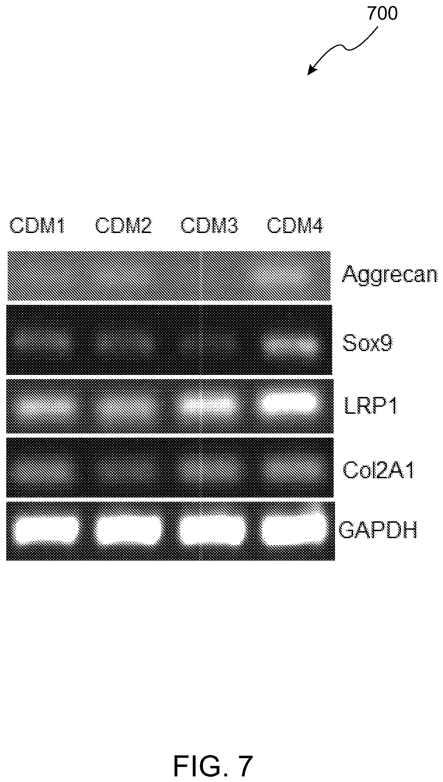

For Reverse transcription PCR (RT-PCR) carried out in FIG. 7, CDM3 which comprises Standard chondrocyte differentiation medium and **500** ug/mL of Chondroitin Sulfate (STD + **5**X CS) and CDM**4** which comprises Standard chondrocyte differentiation medium, Avenanthramide C, and **500** ug/mL of Chondroitin Sulfate (STD + Avn C + 5X CS) are supplemented with **0.1** µM dexamethasone (Sigma-Aldrich #D**4902**) instead of with **0.5** µg/mL hydrocortisone (Sigma-Aldrich #H0**888**) (FIG. 7).

Further, the present disclosure describes the immunofluorescence analysis performed during the experimental stages of the present invention.

On day **21** of chondrocyte differentiation, samples were washed with PBS (Sigma-Aldrich #**56064C**) and fixed with **4**% formaldehyde (Fisher Chemical #F/**1501**) for **10** minutes at room temperature or overnight at **4** °C. This is followed by washing with PBS **3** times for **5** minutes each to remove the fixative. The samples were then permeabilized using **0.1**% Triton-X (Sigma-Aldrich #X**100**) for **15** minutes at room temperature, followed by washing with PBS **3** times for **5** minutes each.

Samples were blocked for at least **1** hour at room temperature or **4**°C overnight with a blocking buffer consisting of **0**.**02**% Tween-**20** (Sigma-Aldrich #P**1379**) and 1% BSA (MP Biomedicals #0**219989880**) in PBS. Samples were then incubated with primary antibodies at **4°C** overnight. Primary antibodies used were Rabbit polyclonal collagen **2** antibodies (Abcam #ab**34712**) and Rabbit monoclonal to SOX9 antibodies (Abcam #ab**185230**), both at a dilution of **1:50** in blocking buffer. Rabbit monoclonal to LRP1 antibodies (Abcam #ab**92544**) was used at a dilution of **1:200** in the blocking buffer. Mouse Anti-Human CD**166** IgG1 Monoclonal Antibody (R&D Systems #SC**017**) was used at a dilution of **1:50.**

Primary antibodies were removed via washing with PBS **3** times for **5** minutes each and then incubated with secondary antibodies for **1** hour at room temperature. The secondary antibody used was Goat polyclonal Secondary Antibody to RabbitIgG - H&L (Alexa Fluor^{®} **555**)(Abcam #ab**150078**) for SOX9, collagen **2,** and LRP1 antibodies and Goat polyclonal Secondary Antibody to Mouse IgG - H&L (Alexa Fluor^{®} **488**)(Abcam #ab**150113**) for CD**166** antibodies, both at a dilution of **1:500** in blocking buffer. Secondary antibodies were removed via washing with PBS **3** times for **5** minutes each and then counter-stained with DAPI (Tocris #**5748/10**). DAPI was removed from the samples by rinsing with PBS. Samples were then stored in PBS at 4°C wrapped in aluminum foil to prevent bleaching of fluorophores. Samples were viewed within 48-72 hours.

Alcian Blue Staining: On day 21 of chondrocyte differentiation, Alcian blue staining was performed as described. Samples were washed with PBS and fixed with 4% formaldehyde for 45 minutes. Following fixation, wells were rinsed twice with DPBS and incubated overnight in 1% Alcian blue stain (pH 2.5), prepared in 0.1N HCl. The next day wells were rinsed three times with 0.1N HCl followed by DPBS to neutralize the acidity. Wells were examined under a light microscope.

### Results:

MSCs exhibit mesenchymal stem cell features and can be effectively induced to adopt chondrocyte-like characteristics.

A comprehensive assessment is conducted to ensure the quality of the mesenchymal stem cells (MSCs), focusing on their retention of both proliferative and differentiative capacities, as well as their expression of MSC markers. Following this verification process, the differentiation of the MSC cells into chondrocyte-like cells is performed, as depicted in FIG. 1A. The cells were treated with a standard chondrocyte differentiation media commonly referred to as STD media.

The results demonstrate the high quality of the MSCs, characterized by their robust proliferative rates and maintenance of MSC markers. Consequently, we successfully induced chondrocyte-like characteristics in our MSCs. FIG. 1A visually outlines the experimental design, depicting the expression of MSC markers at day **0** (D**0**) and their gradual reduction as the differentiation progressed to day **21** (**D21**), as shown in FIG. **1**B. During this transition, the cells exhibited a more rounded morphology, deviating from the fibroblastic appearance typical of MSCs (FIG. 1C).

To confirm the successful chondrocyte differentiation, Alcian blue staining is employed, a widely used technique for detecting cartilage proteoglycans. The staining revealed positive results after **21** days of chondrocyte differentiation (FIG. 1C), providing further evidence of the exceptional quality of our MSCs and their ability to manifest their differentiation potential by acquiring chondrocyte-like features.

FIGS. **1A-1C** illustrate perspective views **100** of key aspects of *in vitro* chondrocyte differentiation process for MSCs, according to one embodiment herein. FIG. 1A presents a schematic diagram outlining the experimental procedure. In FIG. 1B (Scale bar = **50** µm), the expression of MSC markers is depicted at day **0** and day **21** of chondrocyte differentiation using STD media. Furthermore, FIG. 1C (Scale bar = **100** µm) showcases light microscope images of the cells at day **0** and day **21** of chondrocyte differentiation using STD media, accompanied by alcian blue staining.

Further, Avenanthramide C (Avn C) demonstrates superior efficacy in promoting the expression of chondrocyte-specific features compared to the standard MSC-to-chondrocyte differentiation approach.

Chondrocytes are responsible for synthesizing the extracellular matrix (ECM) in cartilage, consisting of large proteoglycans for compressive stiffness and collagen for tensile strength. Among the various collagen types, collagen type 2 (COL2A1) is exclusively found in chondrocytes and constitutes **90-95%** of the collagen present in cartilage ECM. Its role encompasses promoting cell proliferation, ECM deposition, and wound-healing processes within chondrocytes. As a result, COL**2**A**1** is commonly employed as a marker for chondrocyte identification.

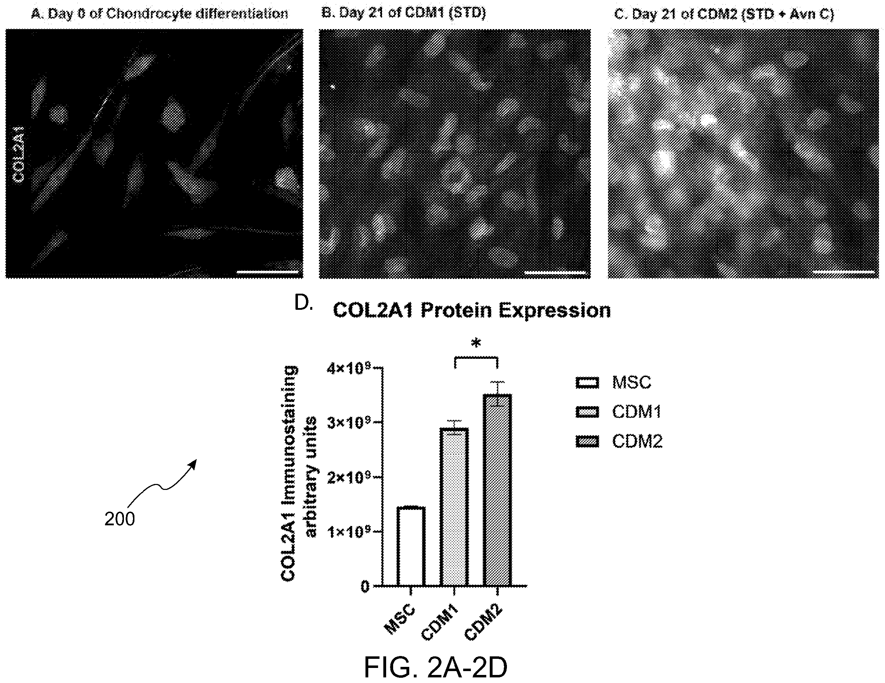

Notably, the introduction of Avenanthramide C (Avn C) enhances chondrocyte differentiation by significantly increasing the expression of the COL**2**A1 protein. This augmentation is evident through the intensified immunofluorescence signals observed in differentiated MSCs following a **21**-day treatment with Avn C in conjunction with the standard differentiation media conditions, referred to as CDM**2** (Avn C). This effect is noticeably more pronounced when compared to cells subjected solely to standard differentiation media, denoted as CDM**1** (STD), as demonstrated in FIG. **2**B and **2**C.

FIGS. **2**A-**2**D illustrate perspective views **200** of Avenanthramides (Avn) C which exerts a potent effect on chondrogenic differentiation by significantly elevating the expression of Collagen **2** protein, according to one embodiment herein. In FIG. **2**A, MSCs are stained for Collagen **2** at day **0** of the MSC-to-chondrocyte differentiation process. FIG. **2**B illustrates MSCs cultured in CDM1 (STD) differentiation media for **21** days. Conversely, in FIG. **2C** (Scale bar = **50** µm), MSCs are cultured in CDM2, which entails treatment with Avn C in addition to the standard differentiation media, for the same duration of **21** days. FIG. **2**D represents the quantification of intracellular COL**2**A1 protein-positive to immunostaining as derived from the intensity and area of immunofluorescent microscopy.

### The impact of dosage range of Avenanthramide C for inducing MSC-to-Chondrocyte Differentiation: A Comprehensive Immunofluorescence Staining Analysis of Collagen 2A1 Expression

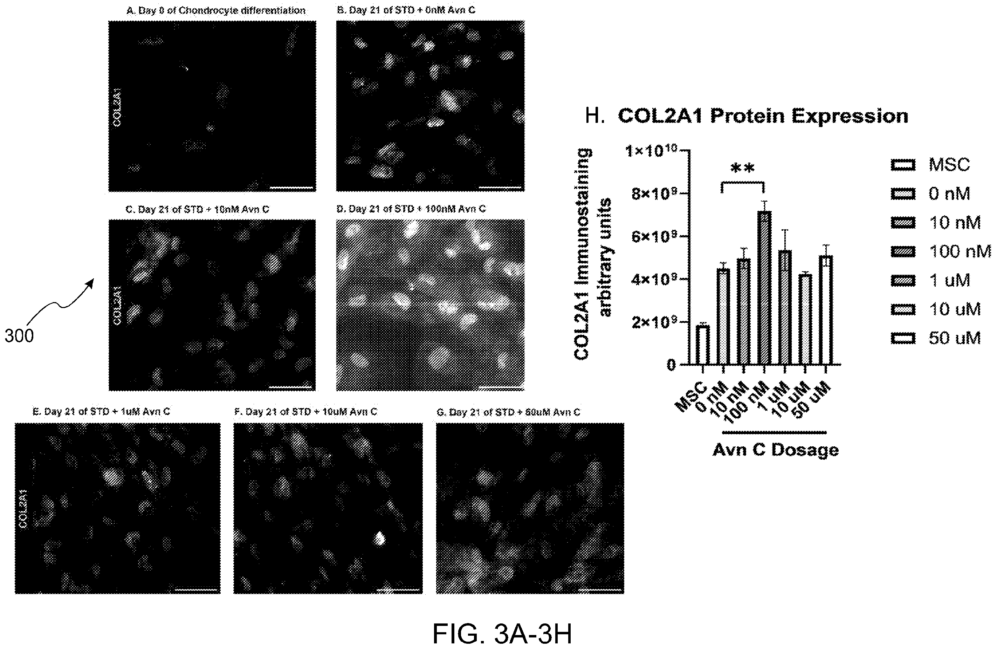

To establish the optimal dosage range of Avenanthramide C (Avn C) in promoting the differentiation of Mesenchymal Stem Cells (MSCs) into chondrocyte-like cells, a meticulous analysis employing immunofluorescence staining of Collagen 2A1 was conducted. FIG. 3 showcases the compelling outcomes of this investigation, wherein FIG. 3A portrays the staining at day **0** (D**0**) when MSCs were initially incubated in differentiation media. Subsequently, MSCs were cultured for 21 days in standard differentiation media supplemented with varying concentrations of Avn C, including 0 nM (FIG. 3B), 10 nM (FIG. 3C), 100 nM (FIG. 3D), 1 uM (FIG. 3E), 10 uM (FIG. 3F), and 50 uM (FIG. 3G). FIG. 3H accentuates the quantitative evaluation of COL2A1 immunological intensity derived from these experimental conditions.

The highly significant results are depicted in FIG. 3 emphasize that the optimal concentration of Avn C for enhancing Collagen 2A1 protein signals resides around 100 nM. Notably, in this experiment, we employed a primary cell line of MSCs from a different individual, further reinforcing the consistency of our observation regarding the impact of Avn C on potentiating the differentiation of MSCs into chondrocyte-like cells, as demonstrated in FIG. **2****.** This dosage range of **10** nM to **50** uM (50,000 nM) of Avn C showcases its remarkable efficacy in driving the differentiation process, as evidenced by the pronounced immunofluorescence staining of Collagen **2**A1.

These findings underscore the powerful potential of Avn C as a transformative agent in facilitating the conversion of MSCs into chondrocyte-like cells, thereby offering promising prospects for regenerative cartilage therapies.

FIGS. **3**A-**3**H illustrate perspective views **300** of identification of the effective dosage range of Avenanthramide C (Avn C) induction in MSC-to-chondrocyte differentiation, in accordance with an embodiment.

FIG. 3A presents immunofluorescence staining of Collagen **2** at day **0** of MSC-to-chondrocyte differentiation.

FIG. 3B presents MSCs cultured in standard chondrocyte differentiation media (STD), supplemented with **0**nM Avn C.

FIG. 3C presents MSCs cultured in standard chondrocyte differentiation media (STD), supplemented with **10**nM Avn C.

FIG. 3D presents MSCs cultured in standard chondrocyte differentiation media (STD), supplemented with **100**nM Avn C.

FIG. 3E presents MSCs cultured in standard chondrocyte differentiation media (STD), supplemented with **1**uM Avn C.

FIG. 3F presents MSCs cultured in standard chondrocyte differentiation media (STD), supplemented with **10**uM Avn C.

FIG. 3G presents MSCs cultured in standard chondrocyte differentiation media (STD), supplemented with **50**uM (50,000 nM) Avn C, for **21** days (Scale bar = **50** µm).

FIG. 3H illustrates the quantification of intracellular COL2A1 protein, determined through the assessment of immunostaining intensity and the corresponding area using immunofluorescent microscopy.

The objective of the present invention is to identify the effective dosage range of Avenanthramide C (Avn C) for inducing MSC-to-chondrocyte differentiation. Immunofluorescence staining of Collagen **2** was performed at day **0** of the differentiation process, providing a baseline comparison. The MSCs were then cultured in standard chondrocyte differentiation media (STD) supplemented with different concentrations of Avn C, including **0**nM, **10**nM, **100**nM, **1**uM, **10**uM, and **50**uM (50,000 nM). The cultures were maintained for **21** days to evaluate the impact of Avn C on chondrocyte differentiation.

### Synergistic Enhancement of Chondrogenic Effects: Avenanthramide C (Avn C) and Chondroitin Sulfate (CS) on MSCs

The chondrogenic effects of Avenanthramide C (Avn C) and Chondroitin Sulfate (CS) on Mesenchymal Stem Cells (MSCs) are demonstrated by their ability to upregulate key proteins involved in chondrocyte differentiation and maturation. Particularly, the transcription factor SOX**9** and the protein LRP1 play pivotal roles in promoting chondrogenic differentiation and regulating the maturity of chondrocytes.

LRP**1** (Low-Density Lipoprotein Receptor-Related Protein **1**) plays a crucial role in chondrocyte maturation, the process by which chondrocytes acquire their mature phenotype and functionality. LRP1 regulates important signaling pathways and extracellular matrix (ECM) components involved in chondrogenesis.

The nuclear localization of SOX**9**, a critical transcription factor, plays a significant role in chondrogenesis, the process by which chondrocytes develop and differentiate into cartilage cells. Proper nuclear localization of SOX**9** is crucial for its transcriptional activity and the activation of chondrogenic gene expression. During chondrogenesis, SOX**9** is translocated into the nucleus, where it binds to specific DNA sequences called enhancer elements within the regulatory regions of chondrogenic genes. This binding activates the transcription of these genes, leading to the synthesis of proteins necessary for chondrocyte function and the formation of cartilage tissue. The nuclear localization of SOX9 is tightly regulated and influenced by various factors, including signaling pathways and co-regulators. These factors modulate the activity and stability of SOX9, ensuring its proper function in chondrogenesis. The nuclear localization of SOX**9** is critical for chondrocyte identity and functionality. It ensures the precise regulation of gene expression necessary for chondrocyte proliferation, extracellular matrix synthesis, and maintaining the characteristics of mature cartilage cells.

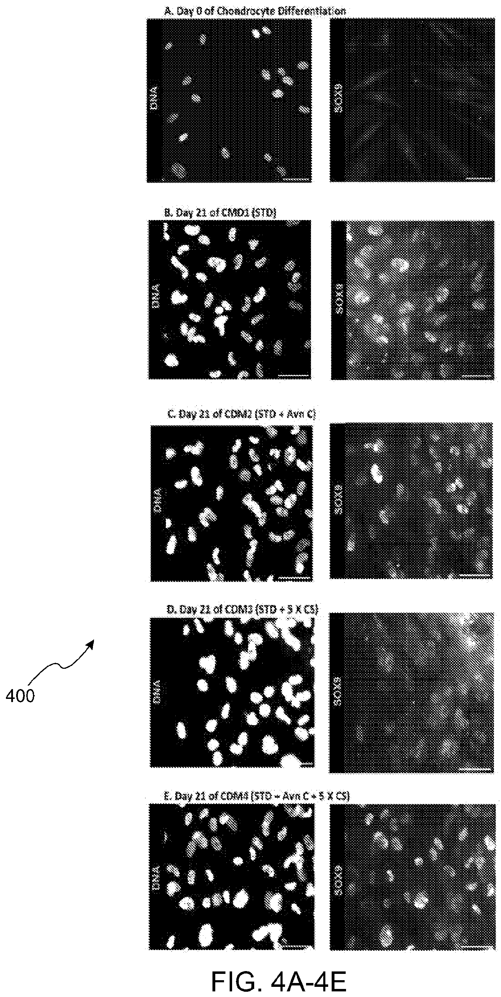

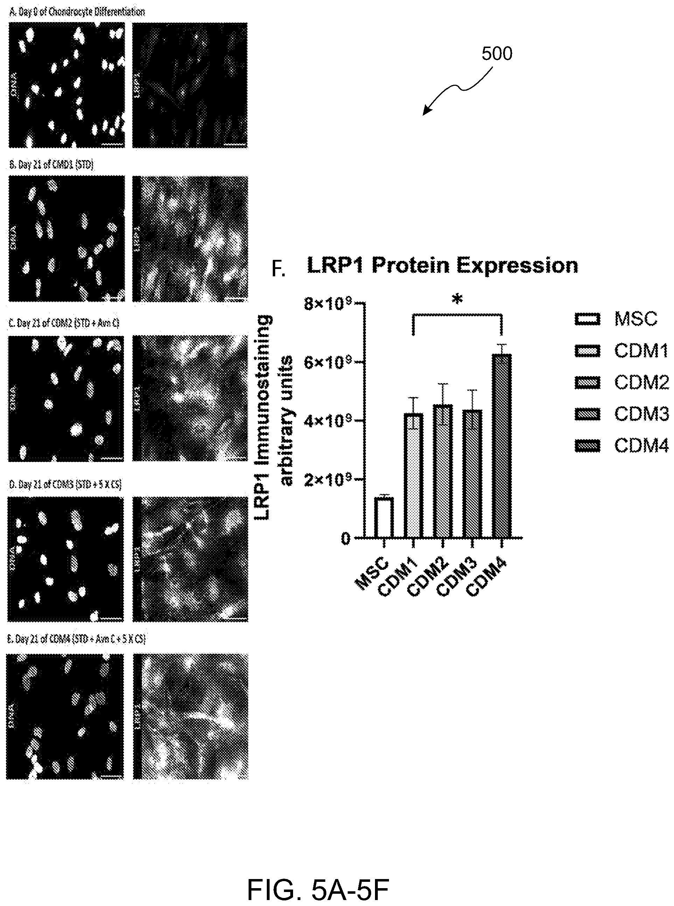

FIGS. **4**A-**4**E illustrate perspective views **400** of the synergistic effects of Avn C with Chondroitin sulfate (CS) on SOX9 protein expression, according to one embodiment herein. FIG. 4A presents immunofluorescent staining of MSCs for SOX**9** at day **0** of MSC-to-chondrocyte differentiation. In FIG. 4B, MSCs were cultured in CDM1 (STD) differentiation media for **21** days. In FIG. 4C, MSCs were cultured in CDM**2** (STD + Avn C) differentiation media for **21** days. In FIG. 4D, MSCs were cultured in CDM**3** (STD + **5**X CS) differentiation media for **21** days. In FIG. 4E, MSCs were cultured in CDM4 (STD + Avn C + **5**X CS) differentiation media for **21** days. FIGS. **4**A-**4**E illustrate the synergistic enhancement of chondrogenic effects achieved by combining Avenanthramide C (Avn C) and Chondroitin Sulfate (CS) on Mesenchymal Stem Cells (MSCs). The figures demonstrate the increased nuclear localization of the transcription factor SOX**9** in FIG. **4** and the upregulation of the protein LRP**1 i**n FIG. **5****,** both of which play vital roles in chondrocyte differentiation and maturation. In FIG. **4****,** MSCs treated with the combined treatment of Avn C and 5X CS (CDM**4**) exhibit intensified immunofluorescence signals for SOX**9** compared to standard differentiation media (CDM1) or CS alone (CDM3). Notably, the immunofluorescent signals in FIG. **4**C and **4**E indicate the presence of functionally active SOX9 with nuclear localization, suggesting its crucial involvement in chondrogenesis when Avn C is present.

Furthermore, the protein LRP1, known for its crucial role in orchestrating chondrocyte functions and maintaining the equilibrium of cartilage tissue, is significantly influenced by the combined treatment of Avn C and CS. LRP1 interacts with growth factors such as TGF-β, IGF-**1**, and PDGF, which are essential for chondrocyte proliferation, differentiation, and ECM synthesis. By modulating the activity of these growth factors, LRP**1** influences the synthesis and organization of ECM components, including collagen and proteoglycans, and contributes to cartilage homeostasis. In addition to ECM regulation, LRP**1** interacts with intracellular signaling molecules and transcription factors, including SOX**9**, RUNX**2**, and Wnt/β-catenin pathway components. These interactions fine-tune gene expression programs involved in chondrocyte function and ECM synthesis.

Immunofluorescence analysis depicted in FIG. **5** provides valuable insights into the expression of LRP**1** during MSC-to-chondrocyte differentiation. FIG. **5**A shows the absence of positive LRP**1** signals in MSCs at the initial stages of differentiation. However, robust immunofluorescence signals for LRP1 are detected in MSCs cultured in CDM**1** (STD) (FIG. **5**B). Remarkably, the most intense LRP**1** immunofluorescence signal is observed in MSCs cultured in CDM**4** (STD + Avn C + **5**X CS), surpassing the signals observed in CDM**3** (STD + **5**X CS) or CDM**2** (STD + Avn C) (FIG. **5**C and **5**D). FIG. **5**F represents quantitative signals of LRP**1** in the described conditions and highlights statistically significant combined treatments of Avn C and CS on LRP**1** protein expressions. These findings unequivocally indicate the synergistic effects of Avn C and **5**X CS in enhancing LRP**1** protein expression, which further promotes chondrocyte differentiation and positively influences their maturity.

Overall, LRP**1** orchestrates chondrocyte functions and maintains cartilage tissue equilibrium. Its role in regulating ECM turnover, growth factor signaling, and transcriptional regulation of chondrogenic genes highlights its significance in chondrocyte maturation. The enhanced expression of LRP1 through the synergistic effects of Avenanthramide C (Avn C) and Chondroitin Sulfate (CS), as demonstrated in our study, further supports LRP**1**'s pivotal role in promoting chondrocyte differentiation and maturation.

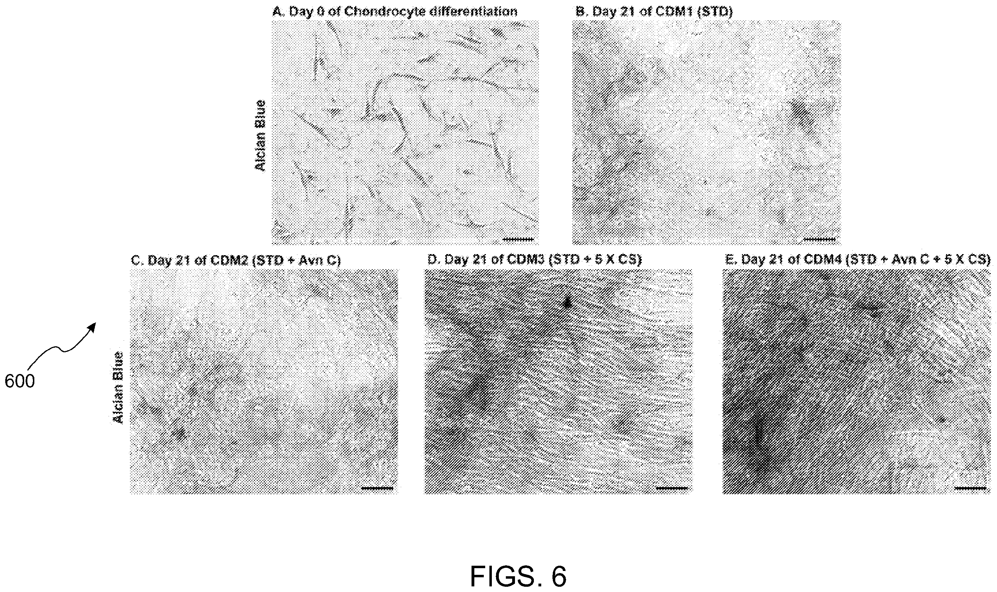

In addition to the increased expression of specific proteins, the presence of specific chondrocyte proteoglycan, as detected by alcian blue staining, confirms the successful differentiation of MSCs into chondrocyte-like cells under the influence of Avn C and CS. Alcian blue staining, a method employed for the detection of cartilage proteoglycans, revealed significant findings in FIG. **6****.** The positive staining observed after treatment with Avn C and CS indicates the successful synthesis and deposition of chondrocyte-specific proteoglycans, reinforcing the chondrogenic potential of the combined treatment. Notably, MSCs cultured in CDM**4** (STD + Avn C + **5** X CS) exhibited the most intense blue staining, surpassing that of CDM3 (STD + **5** X CS) or CDM2 (STD + Avn C) (FIG. **6****).** These results strongly indicate the synergistic effects achieved through the combined application of Avn C and **500** ug/mL of Chondroitin Sulfate (**5**X CS), resulting in enhanced proteoglycan production. This finding underscores the crucial role of proteoglycans in contributing to the compressive stiffness of the cartilage extracellular matrix (ECM).

Collectively, the administration of Avn C and CS enhances the chondrogenic effects on MSCs, as evidenced by the increased nuclear localization of the transcription factor SOX**9** and the expression of protein LRP**1**. The upregulation of SOX9 indicates the induction of chondrogenic differentiation, while the elevated expression of **LRP1** highlights the promotion of chondrocyte maturity. These findings underscore the potential of Avn C and CS as synergistic factors in driving the chondrogenic differentiation of MSCs, making them highly promising candidates for applications in regenerative medicine and tissue engineering strategies focused on cartilage repair and regeneration.

FIGS. **5**A-**5**F illustrate perspective views **500** of synergistic effects of Avn C and Chondroitin Sulfate (CS) on LRP**1** protein expression, in accordance with an embodiment. FIG. 5A presents immunofluorescence staining of LRP**1** in MSCs at day **0** of MSC-to-chondrocyte differentiation. In FIG. 5B, MSCs were cultured in CDM1 (STD) differentiation media for **21** days. In FIG. 5C, MSCs were cultured in CDM**2** (STD + Avn C) differentiation media for **21** days. In FIG. 5D, MSCs were cultured in CDM**3** (STD + **5**X CS) differentiation media for **21** days. In FIG. 5E, MSCs were cultured in CDM**4** (STD + Avn C + **5**X CS) differentiation media for **21** days. The brightest and more evenly distributed LRP**1** immunofluorescence signal is observed in MSCs cultured in CDM**4**, indicating the synergistic effects of Avn C and **500** ug/mL of Chondroitin Sulfate (**5**X CS) on enhancing LRP**1** protein expression. FIG. **5**F displays the quantification of intracellular LRP**1** protein levels, which were evaluated by measuring the immunostaining intensity and the corresponding area using immunofluorescent microscopy.

FIG. **6** illustrates a perspective view **600** of the synergistic effects of Avn C and Chondroitin Sulfate (CS) on Aggrecan protein expression revealed by Alcian blue staining, according to one embodiment herein. FIG. 6A presents Alcian blue staining of MSCs at day **0** of MSC-to-chondrocyte differentiation. In FIG. 6B, MSCs were cultured in CDM1 (STD) differentiation media for **21** days. In FIG. 6C, MSCs were cultured in CDM**2** (STD + Avn C) differentiation media for **21** days. In FIG. 6D, MSCs were cultured in CDM**3** (STD + 5X CS) differentiation media for **21** days. In FIG. 6E, MSCs were cultured in CDM**4** (STD + Avn C + **5**X CS) differentiation media for **21** days. The bluest staining is observed in MSCs cultured in CDM**4**, indicating the enhanced proteoglycan production resulting from the synergistic effects of Avn C and **500** ug/mL of Chondroitin Sulfate (**5**X CS).

### Upregulation of mRNA expressions by Avn C and Chondroitin Sulfate on Chondrocyte Properties: mRNA Analysis via RT-PCR

It is imperative to investigate the impact of Avn C on transcriptionally regulated chondrocyte properties, both when used individually and in conjunction with chondroitin sulfate at an optimal dosage of **500**ug/mL (**5**X CS). To assess this, RT-PCR mRNA expression analysis is employed, which offers precise quantitative data on relative mRNA expressions. Aggrecan (ACAN), a prominent proteoglycan within the cartilage extracellular matrix (ECM) crucial for cartilage repair, serves as a pivotal chondrocyte marker. Aggrecan plays a critical role in the function and integrity of chondrocytes. As a major proteoglycan component of the cartilage extracellular matrix (ECM), it provides essential structural support and contributes to the unique biomechanical properties of cartilage. Aggrecan molecules consist of a core protein and numerous glycosaminoglycan (GAG) chains. The interaction between aggrecan and other ECM components, such as collagen fibers, forms a complex network that maintains the structural integrity of the cartilage tissue. Aggrecan serves as a "water sponge," enabling cartilage to resist compressive forces and maintain its shock-absorbing capacity. This unique property is crucial for the cartilage's ability to withstand mechanical loads and provide cushioning between joints. Imbalances in the aggrecan metabolism and degradation processes have been associated with cartilage degeneration and the development of osteoarthritis.

Remarkably, the intensity of the GAPDH (internal control) bands remains consistent across the CDM**1-4** samples. These findings in FIG. **7** indicate that the differences observed in the expression of Aggrecan, SOX**9**, LRP**1**, and COL**2**A**1** among the samples (CDM**1-4**) are genuine. Specifically, CDM**2** (STD + Avn C) shows a significantly stronger ACAN band compared to CDM1 (STD), providing strong evidence for the effective enhancement of ACAN gene expression by Avn C alone.

Moreover, CDM**4** (STD + Avn C + **5**X CS) displays a more prominent band than CDM3 (**5**X CS) in terms of ACAN expression. This finding underscores the synergistic effects of **5**X CS and Avn C on promoting ACAN gene expression. Strikingly, CDM**4** (STD + Avn C + **5**X CS) exhibits the most intense bands for both SOX**9** and LRP**1**. These compelling results further substantiate the synergistic impact of chondroitin sulfate at an optimal dosage of **500**ug/mL (**5**X CS) and Avn C on augmenting SOX**9** and LRP**1** gene expression. We observed that Avn C treatment alone influences COL**2**A**1** at protein levels (FIGS. **2** and **3****)** rather than at the transcriptional level. Avn C may play a potentiation role in post-translational proteins rather than direct transcription regulation on COL**2**A1.

In summary, the mRNA expression analysis conducted through RT-PCR reinforces the notion that Avn C, when combined with chondroitin sulfate at dosage of **500**ug/mL (**5**X CS), significantly drives MSCs toward adopting chondrocyte properties with enhanced efficacy. The quantitative nature of this analysis provides invaluable insights into the role of Avn C in promoting chondrocyte-specific characteristics.

FIG. **7** illustrates a perspective view **700** of mRNA expression analysis by RT-PCR: Avn C exhibits synergistic effects with Chondroitin sulfate (CS) for Aggrecan (ACAN), SOX9, and LRP**1** gene expression, while maintaining COL**2**A1 gene expression, according to one embodiment herein. The expression levels of the target genes were assessed using RT-PCR in MSCs cultured in different media conditions. The combination of Avn C and CS enhances the expression of ACAN, SOX**9**, and LRP**1** genes, indicating their synergistic effects on chondrocyte-related gene expression. Additionally, the expression of the COL**2**A1 gene is maintained, suggesting the preservation of chondrocyte-specific characteristics. GAPDH was used as an internal control to ensure accurate normalization of the mRNA expression levels among the samples.

### Consistent Enhancement of Chondrocyte Differentiation in MSCs from Multiple Biological Samples through the Combined Action of Avn C and Chondroitin Sulfate

To further validate and reinforce the efficacy of our innovative approach, which involves the utilization of Avn C in combination with a high concentration of chondroitin sulfate at **500**ug/mL (5X CS), we conducted extensive experimentation using multiple biological samples. The objective was to investigate the reproducibility and consistency of the observed effects on adult mesenchymal stem cells (MSCs) when subjected to the chondrocyte differentiation protocol.

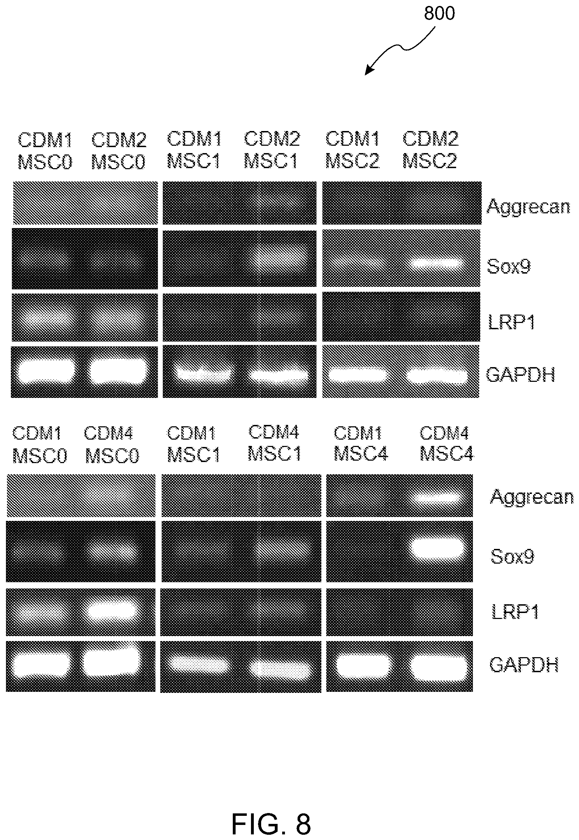

To evaluate the robustness of our method, we implemented the CDM**4** (STD + Avn C + **5**X CS) differentiation media on MSCs obtained from different individuals. This experimental design allowed us to assess the performance of our approach across various biological samples, ensuring that our findings were not limited to a specific cell source (FIG. **8****.)**

Remarkably, the results of our investigation consistently demonstrated the enhancement of chondrocyte differentiation in response to the Avn C and chondroitin sulfate combination. The observed effects were reproducible and maintained a high level of consistency across all the tested biological samples. This validation provides strong evidence supporting the effectiveness and reliability of the approach in driving MSCs toward a chondrocyte-like phenotype.

By utilizing multiple biological samples that came from different ages and genders, we were able to ascertain that the positive outcomes were not dependent on individual variations or experimental anomalies. The consistent enhancement of chondrocyte differentiation across diverse cell sources further reinforces the potential translational application of our method in regenerative medicine and cartilage tissue engineering.

In summary, the comprehensive validation of our concept involving the use of Avn C in combination with a high concentration of chondroitin sulfate, conducted on adult MSCs obtained from different individuals, unequivocally confirms the reproducibility and reliability of our approach. These findings establish a solid foundation for future research and the development of advanced strategies to effectively promote chondrocyte differentiation and facilitate cartilage regeneration.

FIG. **8** illustrates a perspective view **800** of mRNA expression analysis of chondrocyte markers in multiple individuals' MSCs using RT-PCR, according to one embodiment herein. The impact of Avn C alone (namely CDM2 compared to CDM1) as well as the effects of Avn C in combination with **500** ug/mL (5X) of Chondroitin sulfate (CS) treatment (notably CDM4 compared to CDM1) on gene expression levels of AGGRECAN, SOX9 and **LRP1,** relative to GAPDH, in adult MSCs obtained from different individuals: MSC**0**, MSC**1**, MSC**2**, and MSC**4**. The results demonstrate significantly increased gene expressions of AGGRECAN, SOX9 and **LRP1** in response to the Avn C and 5X CS treatment. Notably, these findings consistently hold across multiple MSC lines, indicating the reproducibility and reliability of the observed effects.

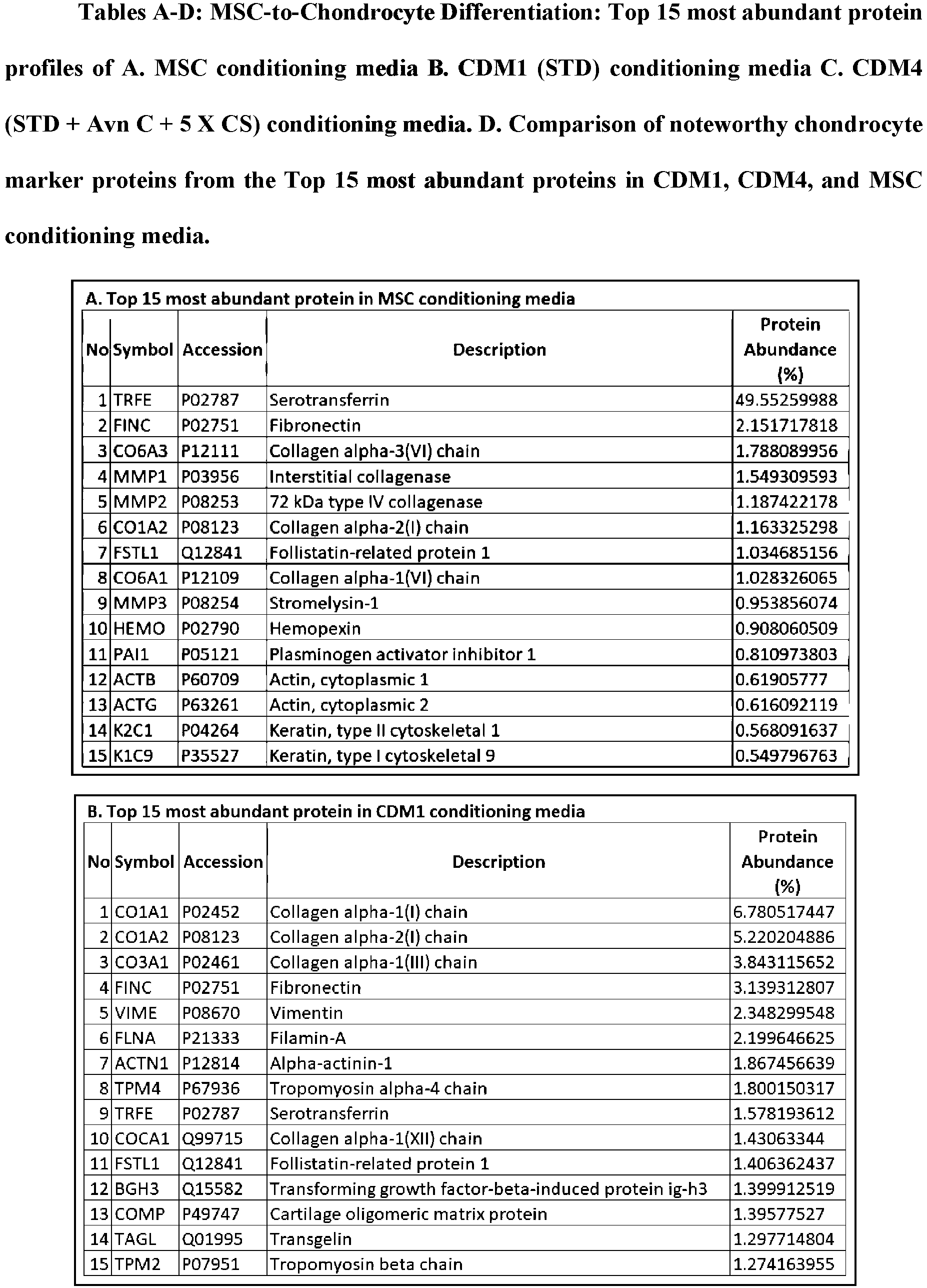

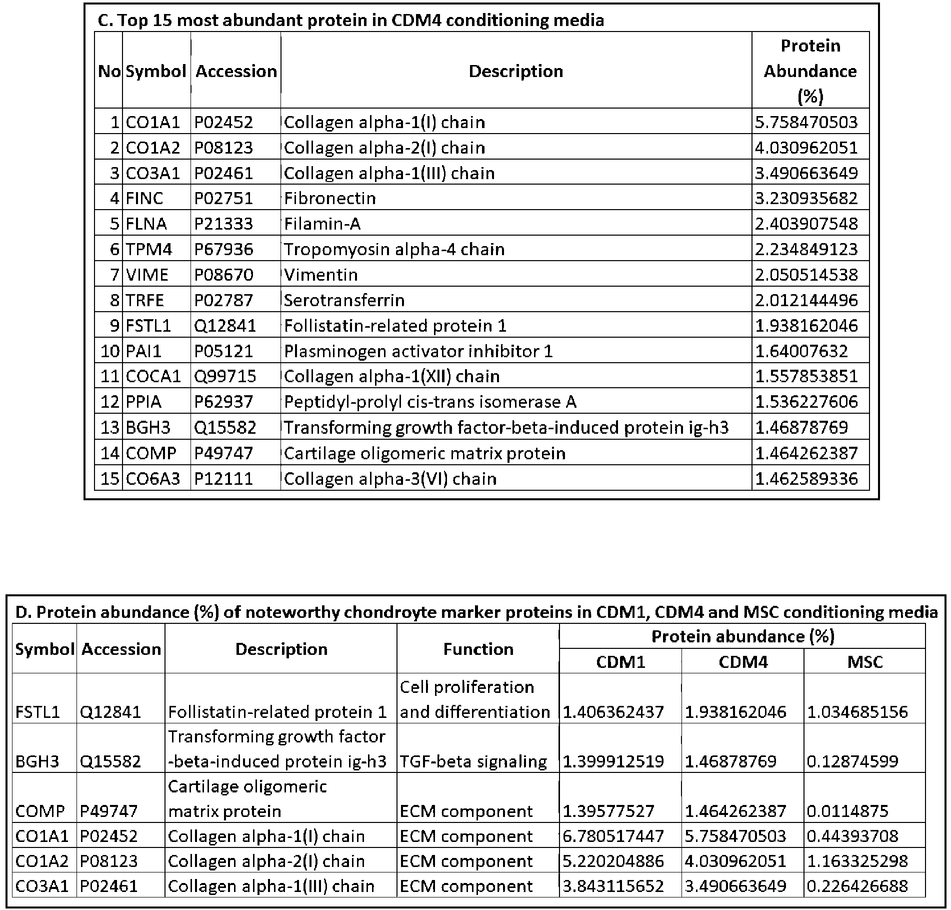

### Profiling the Secretome: Unveiling the Functional Protein Landscape of MSC-to-Chondrocyte Differentiation Conditioned Media and Validation of the Avn C and Chondroitin Sulfate Approach.

Tables A-D above provide a comprehensive analysis of the secreted protein profiles in different treatment groups, emphasizing the significance of validating chondrocyte functions in cartilage repair. A detailed breakdown is presented, showcasing the top **15** most abundant proteins in the conditioning media of MSCs, CDM1 (STD), and CDM**4** (STD + Avn C + **5**X CS). Notably, the presence of chondrocyte marker proteins among the identified proteins in CDM1 and CDM4 is crucial for confirming the functional behavior of differentiated cells resembling native chondrocytes and their ability to contribute to cartilage repair. Secreted proteins play a vital role in validating chondrocyte functions in cartilage repair. Chondrocytes are essential for cartilage development and maintenance, and the identification of chondrocyte marker proteins is crucial for monitoring and validating the differentiation of MSCs toward a chondrocyte-like phenotype. By comparing the protein profiles, we gain insights into the distinctive characteristics of MSCs, CDM1, and CDM4 conditioning media. This comprehensive analysis allows us to identify proteins that are particularly enriched in each condition, shedding light on the key players involved in the differentiation process.

Furthermore, the validation of chondrocyte marker proteins among the treatment groups provides valuable information regarding the effectiveness of the Avn C and chondroitin sulfate combination for cartilage repair. Examining the secreted proteins allows us to assess the impact of Avn C and CS treatment not only on intracellular gene and protein markers but, more importantly, on the secreted proteins, which signify the ability of differentiated cells to function successfully, similar to native chondrocytes.

The presence and abundance of these markers in CDM**1** and CDM**4** conditioning media indicate the successful induction of MSCs toward a chondrocyte-like state. By delving into the proteomic landscape, we gain insights into the protein profiles associated with MSC differentiation and validate the expression of chondrocyte marker proteins. This thorough analysis not only enhances our understanding of the underlying mechanisms driving MSC-to-chondrocyte differentiation but also reinforces the notion of using Avn C in combination with high concentrations of chondroitin sulfate for more efficient and effective differentiation outcomes.

Tables A-C outline the protein profiles of MSC, CDM1, and CDM**4** conditioning media, respectively, highlighting the top **15** most abundant proteins. Notably, chondrocyte markers are selected from the top **15** proteins identified in CDM1 and CDM**4** conditioning media. One such marker is Follistatin-related protein **1** (FSTL-1), which is found in both MSCs and cartilage but exhibits significantly higher abundance in CDM**4**-treated cells. FSTL-1 has been implicated in regulating chondrocyte proliferation, MSC-to-chondrocyte differentiation, and the expression of cartilage extracellular matrix (ECM) proteins. Table D demonstrates the upregulation of FSTL-1 protein in the conditioned media of MSCs treated with CDM**1** and CDM**4** compared to MSC-conditioned media alone, thereby validating FSTL-**1** as a potent regulator of MSC-to-chondrocyte differentiation. Additionally, the higher abundance of FSTL-1 in CDM**4**-conditioned media compared to CDM1-conditioned media provides evidence that the combined treatment of Avn C and **500** ug/mL of Chondroitin sulfate (**5**X CS) is more effective in driving MSC-to-chondrocyte differentiation. Transforming growth factor-beta (TGF-β) superfamily BGH**3**, which induces in vitro MSC-to-chondrocyte differentiation, and ECM proteins such as cartilage oligomeric matrix protein (COMP), Collagen alpha-**1** (I) (CO**1**A**1**), Collagen alpha-**2** (I) (CO1A**2**), Collagen alpha-**1** (III) (CO**3**A1), and COMP, which are commonly found in the chondrocyte secretome, serve as hallmark genes of chondrocytes. The higher protein abundance of these markers in the conditioned media of MSCs treated with CDM1 and CDM**4** compared to MSC-conditioned media alone confirms the successful differentiation of MSCs into chondrocytes.

The presence and abundance of chondrocyte marker proteins in the conditioned media of MSCs treated with CDM1 and CDM**4** suggest successful induction of MSCs towards a chondrocyte-like state. This validation of chondrocyte functions is critical in the context of cartilage repair, as the secretion of specific proteins indicates the ability of differentiated cells to contribute to cartilage regeneration and maintenance. By exploring the secreted protein landscape, we unravel the proteomic profiles associated with MSC-to-chondrocyte differentiation and validate the presence of chondrocyte marker proteins. This comprehensive analysis not only enhances our understanding of the underlying mechanisms driving MSC-to-chondrocyte differentiation but also reinforces the importance of utilizing Avn C in conjunction with high concentrations of chondroitin sulfate for more efficient and effective differentiation outcomes.

Overall, the analysis presented in Tables A-D provide a comprehensive snapshot of the secreted protein profiles in different treatment groups, confirming that the differentiated cells were able to secrete proteins important in chondrocyte functions for cartilage repair. The identification and validation of chondrocyte marker proteins emphasize the significance of evaluating the functional protein landscape to assess the success of MSC-to-chondrocyte differentiation and to ensure their potential for contributing to cartilage regeneration and repair.

The embodiments herein provide a composition for inducing differentiation of mesenchymal stem cells (MSCs) into chondrocytes, said composition comprising Avenanthramide C (Avn C). and chondroitin sulfate.

It is to be understood that the phraseology or terminology employed herein is for the purpose of description and not of limitation. Therefore, while the embodiments herein have been described in terms of preferred embodiments, those skilled in the art will recognize that the embodiments herein can be practiced with modifications.

## Claims

1. A composition for inducing differentiation of mesenchymal stem cells (MSCs) into chondrocytes, said composition comprising Avenanthramide C (Avn C) and chondroitin sulfate (CS) as a bioactive factor, wherein said composition is a medium or hydrogel.

2. The composition according to claim 1, wherein said Avenanthramide C (Avn C) is present at the concentration in the range of 10 nM - 50,000 nM, preferably at a concentration of 50 - 300 nM.

3. The composition according to claim 2, wherein said chondroitin sulfate (CS) is present at the concentration in the range of 300 - 700 µg/mL.

4. The composition according to claim 1, wherein said mesenchymal stem cells are adipose-derived mesenchymal stem cells (aMSCs).

5. The composition according to any one of claims 1-4, wherein said chondrocytes are 2-dimensional manner chondrocyte-like cells.

6. The composition according to any one of claims 1-4, for increasing chondrocyte markers in said chondrocytes, and wherein said chondrocyte markers are selected from the group consisting of FSTL-1, LRP1, ACAN, COL2A, SOX9, and any combination thereof.

7. Use of a composition according to anyone of claims 1 to 4 for inducing *in vitro* differentiation of mesenchymal stem cells (MSCs).

8. A method for inducing differentiation of *in vitro* cultured mesenchymal stem cells (MSCs) into chondrocytes, said method comprising a step of culturing said MSCs in a medium comprising Avenanthramide C (Avn C) and chondroitin sulfate (CS) as a bioactive factor.

9. The method according to claim 8, wherein said Avenanthramide C (Avn C) is present at the concentration in the range of 10 nM - 50,000 nM, preferably present at the concentration in the range of 50 nM - 300 nM.

10. The method according to claim 9, wherein said chondroitin sulfate (CS) is present in the medium at the concentration in the range of 300 - 700 µg/mL.

11. The method according to claim 8, wherein said mesenchymal stem cells are adipose-derived mesenchymal stem cells (aMSCs).

12. The method according to any one of claims 8 to 11, wherein said chondrocytes are 2-dimensional manner chondrocyte-like cells.

13. The method according to any one of claims 8 to 11, wherein chondrocyte markers of said chondrocytes are increased, and wherein said chondrocyte markers are selected from the group consisting of FSTL-1, LRP1, ACAN, COL2A, SOX9, and any combination thereof.

## Patentansprüche

1. Zusammensetzung zur Induktion der Differenzierung von mesenchymalen Stammzellen (MSCs) in Chondrozyten, wobei die genannte Zusammensetzung Avenanthramid C (Avn C) und Chondroitinsulfat (CS) als bioaktiven Faktor umfasst, wobei die genannte Zusammensetzung ein Medium oder ein Hydrogel ist.

2. Zusammensetzung nach Anspruch 1, wobei das genannte Avenanthramid C (Avn C) in einer Konzentration im Bereich von 10 nM - 50.000 nM, vorzugsweise in einer Konzentration von 50 - 300 nM, vorliegt.

3. Zusammensetzung nach Anspruch 2, wobei das genannte Chondroitinsulfat (CS) in einer Konzentration im Bereich von 300 - 700 µg/mL vorliegt.

4. Zusammensetzung nach Anspruch 1, wobei die genannten mesenchymalen Stammzellen aus adipös-abgeleiteten mesenchymalen Stammzellen (aMSCs) sind.

5. Zusammensetzung nach einem der Ansprüche 1-4, wobei die Chondrozyten zweidimensional-artige chondrozytenähnliche Zellen sind.

6. Zusammensetzung nach einem der Ansprüche 1 bis 4, zur Erhöhung von Chondrozytenmarkern in den genannten Chondrozyten, und wobei die genannten Chondrozytenmarker aus der Gruppe ausgewählt sind, die aus FSTL-1, LRP1, ACAN, COL2A, SOX9 und beliebigen Kombinationen davon besteht.

7. Verwendung einer Zusammensetzung gemäß einem der Ansprüche 1 bis 4 zur Induktion einer in-vitro-Differenzierung von mesenchymalen Stammzellen (MSCs).

8. Verfahren zur Induktion der Differenzierung von *in vitro* kultivierten mesenchymalen Stammzellen (MSCs) in Chondrozyten, wobei das genannte Verfahren einen Schritt der Kultivierung der genannten MSCs in einem Medium umfasst, das Avenanthramid C (Avn C) und Chondroitinsulfat (CS) als bioaktiven Faktor umfasst.

9. Verfahren nach Anspruch 8, wobei das genannte Avenanthramid C (Avn C) in einer Konzentration im Bereich von 10 nM - 50.000 nM, vorzugsweise in einer Konzentration im Bereich von 50 nM - 300 nM, vorliegt.

10. Verfahren nach Anspruch 9, wobei das genannte Chondroitinsulfat (CS) in dem Medium in einer Konzentration im Bereich von 300 - 700 µg/mL vorliegt.

11. Verfahren nach Anspruch 8, wobei die genannten mesenchymalen Stammzellen aus adipös-abgeleiteten mesenchymalen Stammzellen (aMSCs) sind.

12. Verfahren nach einem der Ansprüche 8 bis 11, wobei die genannten Chondrozyten zweidimensional-artige chondrozytenähnliche Zellen sind.

13. Verfahren nach einem der Ansprüche 8 bis 11, wobei Chondrozytenmarker der genannten Chondrozyten erhöht sind, und wobei die genannten Chondrozytenmarker aus der Gruppe ausgewählt sind, die aus FSTL-1, LRP1, ACAN, COL2A, SOX9 und beliebigen Kombinationen davon besteht.

## Revendications

1. Composition pour induire la différenciation de cellules souches mésenchymateuses (MSC) en chondrocytes, ladite composition comprenant de l'avénanthramide C (Avn C) et du sulfate de chondroïtine (CS) en tant que facteur bioactif, laquelle composition est un milieu ou un hydrogel.

2. Composition selon la revendication 1, dans laquelle ledit avénanthramide C (Avn C) est présent à une concentration située dans la plage allant de 10 nM à 50 000 nM, de préférence à une concentration de 50 à 300 nM.

3. Composition selon la revendication 2, dans laquelle ledit sulfate de chondroïtine (CS) est présent à une concentration située dans la plage allant de 300 à 700 µg/ml.

4. Composition selon la revendication 1, dans laquelle lesdites cellules souches mésenchymateuses sont des cellules souches mésenchymateuses dérivées de tissu adipeux (aMSC).

5. Composition selon l'une quelconque des revendications 1 à 4, dans laquelle lesdits chondrocytes sont des cellules analogues à des chondrocytes de manière bidimensionnelle.

6. Composition selon l'une quelconque des revendications 1 à 4, pour augmenter les marqueurs de chondrocytes dans lesdits chondrocytes, et dans laquelle lesdits marqueurs de chondrocytes sont choisis dans le groupe constitué par FSTL-1, LRP1, ACAN, COL2A, SOX9, et l'une quelconque de leurs combinaisons.

7. Utilisation d'une composition selon l'une quelconque des revendications 1 à 4, pour induire la différenciation *in vitro* de cellules souches mésenchymateuses (MSC).

8. Procédé pour induire la différenciation de cellules souches mésenchymateuses cultivées *in vitro* (MSC) en chondrocytes, ledit procédé comprenant une étape de culture desdites MSC dans un milieu comprenant de l'avénanthramide C (Avn C) et du sulfate de chondroïtine (CS) en tant que facteur bioactif.

9. Procédé selon la revendication 8, dans lequel ledit avénanthramide C (Avn C) est présent à une concentration située dans la plage allant de 10 nM à 50 000 nM, de préférence présent à une concentration située dans la plage allant de 50 à 300 nM.

10. Procédé selon la revendication 9, dans lequel ledit sulfate de chondroïtine (CS) est présent dans le milieu à une concentration située dans la plage allant de 300 à 700 µg/ml.

11. Procédé selon la revendication 8, dans lequel lesdites cellules souches mésenchymateuses sont des cellules souches mésenchymateuses dérivées de tissu adipeux (aMSC).

12. Procédé selon l'une quelconque des revendications 8 à 11, dans lequel lesdits chondrocytes sont des cellules analogues à des chondrocytes de manière bidimensionnelle.

13. Procédé selon l'une quelconque des revendications 8 à 11, dans lequel des marqueurs de chondrocytes desdits chondrocytes sont augmentés, et dans lequel lesdits marqueurs de chondrocytes sont choisis dans le groupe constitué par FSTL-1, LRP1, ACAN, COL2A, SOX9, et l'une quelconque de leurs combinaisons.
